# EUROPEAN PATENT SPECIFICATION

(11) **EP 1 088 810 B1**
(45) Date of publication and mention of the grant of the patent: **20.07.2005**
(21) Application number: 99307767.6
(22) Date of filing: 01.10.1999
(51) Int. Cl.: C07C 45/36, C07C 47/54

(54) **Process for the production of benzaldehyde by the catalytic liquid phase air oxidation of toluene**
Verfahren zur Herstellung von Benzaldehyd durch katalytische Luftoxidation von Toluol in der Flüssigphase
Procédé de préparation de benzaldéhyde par oxidation catalytique du toluène par air en phase liquide

(43) Date of publication of application: 04.04.2001
(73) Proprietor: Council of Scientific and Industrial Research, New Delhi 110 001 (IN)
(72) Inventor: Kantam, Mannepalli Lakshmi, Andhra Pradesh (IN); Choudary, Boyapati Manoranjan, Andhra Pradesh (IN); Sreekanth, Pentlavally, Andhra Pradesh (IN); Rao, Kottapalli Koteswara, Andhra Pradesh (IN); Naik, Kantarao, Andhra Pradesh (IN); Kumar, Thella Prathap, Andhra Pradesh (IN); Khan, Asad Ali, Andhra Pradesh (IN)
(74) Representative: Manaton, Ross Timothy

(56) References cited:
- CH-A- 645 335
- DATABASE WPI Section Ch, Week 197809 Derwent Publications Ltd., London, GB; Class E14, AN 1978-16645A XP002134845 & JP 53 005132 A (MITSUBISHI CHEM IND LTD) , 18 January 1978 (1978-01-18)
- H.V. BORGAONKAR ETAL.: "Liquid Phase Oxidation of Toluene to Benzaldehyde by Air" IND. ENG. CHEM. PROD. RES. DEV., vol. 23, 1984, pages 455-458, XP000882265
- T. OKADA: "The Liquid-phase Oxidation of Methylbenzenes by the Cobalt-Copper-Bromine system" BULL. CHEM. SOC. JPN., vol. 54, no. 9, 1981, pages 2724-2727, XP000882017
- T.I.A. GERBER ET AL.: "The partial air oxidation..." S. AFR. J. CHEM., vol. 51, no. 4, 1998, pages 178-185, XP000882018

## Description

### Field of the invention

The present invention relates to a process for the production of benzaldehyde by catalytic liquid phase air oxidation of toluene, preferably with 40-50% selectivity.

The N.F./F.C.C. grade of benzaldehyde is widely used in flavors such as almond and cherry and in various fragrances for soap and toiletries. Benzaldehyde is a F.D.A. sanctioned synthetic flavoring substance generally recognized as safe for food. The technical grade is a versatile chemical intermediate in the manufacture of pharmaceuticals, dyes, perfume and flavoring chemicals. The technical grade of benzoic acid is used as an intermediate in the manufacture of chemicals, alkyd resins, polyesters, plasticizers, dyestuffs, preservatives, rubber activators and retardants. Benzoic acid, industrial grade, is used as a chemical intermediate and as a diverting agent in crude oil recovery applications .

### Background of the invention

Benzaldehyde is currently produced by the hydrolysis of the corresponding side chain halogenated toluene compound. US Patent 4,229,379, Oct 21, 1980 describes the preparation of benzaldehyde by hydrolysis of benzyl chloride at 100-200°C at normal or under increased pressures in the presence of an excessive aqueous hydrochloric acid. US Patent 4,450,298, May 22, 1994, discloses vapour phase catalytic hydrolysis of benzyl chloride to form benzaldehyde by using activated carbon treated with H₂SO₄ or impregnated with a metal chloride such as FeCl₃ or a metal sulphate such as cupric sulphate. The drawbacks in the above processes are generation of large excess of effluents and the benzaldehyde produced does not meet food grade specifications.

Air oxidation of toluene and its derivatives offers green technology path, provided the desired selectivities are realised for market driven products and minimisation of halogenated and unwanted by-products causing effluents is achieved. Several patents and or applications describe innovation in air oxidation of toluene and its derivatives both in liquid and vapour phase.

One of the prevalent industrial practices for the vapour phase oxidation of toluene to benzaldehyde involves a uranium oxide/molybdenum oxide catalyst at 500-600°C (W L. Faith, D.B. Keyes and R.L. Clark, Industrial Chemicals, 3^{rd} Ed., John Wiley & Sons, Inc., New York, 1965); (US Patent No. 3,579,589). US Patent 3,946,067 discloses a process for the preparation of aromatic aldehydes such as benzaldehyde or substituted benzaldehydes by the vapour-phase oxidation of aralkyl compounds, such as toluene or substituted toluenes, respectively, at temperatures of less than ∼ 250°C in the presence of a catalyst composition containing phosphoric acid and a catalytically effective amount of palladium metal. The aromatic aldehydes are produced in a single reaction step. The drawbacks in this process are that the conversion of toluene has to be kept very low < 4% to attain high selectivity (>70%) of benzaldehyde and significant amount of carbon dioxide is also formed in this process.

US Patent 3,989,674, Nov 2, 1976 describes a process wherein a mixture of toluene, oxygen and a helium diluent in molar ratio 1:2:8 is passed over the Cu-Au-silica catalyst at atmospheric pressure and temperatures in the range of 450-600°F with 200-1000 volumes of gas/h/volume of catalyst. The selectivity of benzaldehyde is 75-80% at conversion levels 15-30%. US Patent 4,137,259, Jan 30, 1979, describes a process for the vapour phase catalytic oxidation of toluene to benzaldehyde and benzoic acid at a temperature ranging from 300-500°C in the presence of a silver-iron vanadate on silica with conversion 21%. A selectivity to aldehyde of 33% is described. US Patent 4,390,728, June 28, 1983, describes a process wherein benzaldehyde is formed by the oxidation of toluene in the presence of a catalyst composed of the oxides of Cu, Fe, Pb, U, Mo, and P, and optionally including some other promoter elements. The reaction conditions are 475-550°C, 0-10 atm. pressure, per pass conversion is 35-50%, and selectivity to benzaldehyde 40-70%. Ray et al, Ind. J. Technol., 21(4), 137, 1983, reported a process for the oxidation of toluene to benzaldehyde. But, the conversion per pass is restricted to ~15% and the yield of benzaldehyde is generally not more than 70%, with CO₂ as main product Further, the low concentration of toluene in the toluene-air feed mixture poses problems of recovery. The drawbacks in the above processes are the use of higher reaction temperatures, generation of carbon dioxide in large amounts contributing to global warming. Therefore, these processes do not appear to be attractive.

US Patent 5,476,827, December 19, 1995, discloses the preparation of aldehydes by the reduction of acids and esters in vapour phase in the presence of a bimetallic catalyst. The drawback is the two-step process of oxidation of toluene, a desired raw material, to benzoic acid and reduction of benzoic acid to benzaldehyde with hydrogen. Eventually, this process becomes uneconomical, when compared to a process of selective oxidation of toluene to benzaldehyde conceived.

Morimoto et al (J. Chem. Soc. Sect. B, 62, 1967), Fields et al (Adv. Chem. Ser., No. 76 (2), 395) and Kamiya (Adv. Chem. Ser., No. 76 (2), 193, 1968) have reported that liquid phase air oxidation provides high yield of benzaldehyde when oxidation is carried out in acetic acid medium with cobalt acetate as catalyst and sodium bromide as promoter. The drawbacks are that this process suffers from the disadvantages of relatively low yield. US Patent 2,959,613, describes a process wherein the liquid phase oxidation of toluene or its nucleus substituted materials, such as xylene, is carried out by oxygen under the presence of a catalyser containing a bromine compound and a heavy metallic compound (such as a cobalt compound or manganese compound) along with a zinc compound or an alkaline earth metallic compound or an alkaline metallic compound. The drawbacks in this method are that the main product is the corresponding aromatic carbonic acid and either there is absolutely no production of the corresponding aromatic aldehyde or it is produced in a very small quantity as a by-product.

Japanese Patent No. SHO-53-5132 discloses a process wherein in order to increase the selectivity of benzaldehyde or its nucleus substituted material, a large quantity of catalyser containing a cobalt compound and bromine compound is used. Japanese Patent No. SHO-56-108728, August 28,1981, describes a process wherein the liquid phase air oxidation of toluene is carried out by a catalyst comprising a heavy metallic compound, zinc and bromine compound at 30-180°C and a small pressure. The transformation percentage of toluene is maintained within a specific range with the advantage of the execution of the reaction employing carboxylic acid as solvent in the range 0.5-2.0 times with respect to toluene or its substituted material. By this method, the selectivity of benzaldehyde is increased while formation of benzyl bromide is reduced to 2 mol%. However, the turnover number is 3-50 in these air oxidation reactions. The drawback in the above processes are that low turnover numbers render the process uneconomical; the use of higher catalyst concentration hasten corrosion of reactors and excessive production of benzyl bromide is detrimental to achieve the desired quality of the product. US Patent 3,969,405 wherein the oxidation of toluene in the presence of cobalt acetate, acid activator and molecular oxygen oxidant giving high yield of benzoic acid with selectivity to benzaldehyde 35% is disclosed. US Patent 5,473,101 describes a process wherein the oxidation of toluene is carried out in the presence of cobalt acetate, sodium bromide and hydrogen peroxide disclosing conversion of 90.6%, benzaldehyde yield of 29.0%, benzoic acid yield of 55.6%. There are drawbacks in the above processes due to excessive production of benzyl bromide or its nucleus substituted material. Therefore, such a method cannot be said to be a satisfactory one from the point of view of industrial production Swiss Patent Application No. 645 335 A also teaches the use of molecular oxygen as an oxidant, the disadvantages of which have been discussed above.

Borgaonkar et al. (Ind. Eng. Chem. Prod. Res. Dev., Vol. 23, 1984, p 455-458) teaches the liquid phase oxidation of toluene by air in acetic acid medium, with cobalt acetate catalyst and sodium bromide promoter. However, the catalyst and the promoter are required in large amounts and the reaction time is long.

Okada (Bull. Chem. Soc. Jpn., Vol. 54, 1981, p. 2724-2727) teaches the liquid phase oxidation of methylbenzenes by a catalyst system comprising cobalt (II) and copper (II) acetates and sodium bromide in acetic acid at high temperature and high pressure. The process also requires a high concentration of catalyst, the disadvantages of which are discussed above.

Gerber et al. (S. Afr. J. Chem., Vol. 51, No. 4, 1998, p. 178-185) teaches the partial air oxidation of alkyl aromatic compounds by cobalt (II) acetate and bromide in carboxylic acid solvents. The use of catalyst is not disclosed.

Obviously different approaches have been employed both at laboratory and commercial scale to prepare benzaldehyde. Traditional process has the disadvantage of forming unwanted and corrosive halogenated by-products.

### Objects of the invention

The main object of the present invention is to provide an improved process for the production of benzaldehyde with 40-50% selectivity by the catalytic liquid phase air oxidation of toluene.

Another object of the present invention is maintaining transformation percentage <25% in the catalytic liquid phase air oxidation of toluene.

Yet another object of the present invention is the production of benzaldehyde (40-50%) in very low concentration of catalyst thereby rendering the process economical.

Yet another object of the present invention is the production of benzyl alcohol (5-10%) another value added product of toluene in very low concentration of catalyst rendering the process economical.

Yet another object of the invention is to restrict the conversion to obtain high selectivity of benzaldehyde by the termination of the reaction after 1-1.5hrs.

Yet another object of the present invention is to achieve production of benzaldehyde with a low concentration of catalyst, co-catalyst and promoter thus slowing down corrosion of the reactor which is a very important factor affecting economics of the project.

Yet another object of the present invention is to obtain near identical conversions and selectivities without the addition of promoter/bromine source in recycle reactions conducted with the recovered composite mixture of catalyst, co-catalyst and promoter.

Yet another object of the present invention is to minimise the consumption of bromide promoter in recycle reactions.

Yet another object of the invention is to eliminate the formation of benzyl bromide which is detrimental to the quality of benzaldehyde.

Yet another object of the present invention is to provide a process where the turnover number is in the range of 350-700.

Yet another object of the present invention the benzaldehyde produced is chlorine free and can be used for a wide range of applications.

Another object of the present invention is to provide an environmentally safe process by elimination of the effluent disposal problem.

### Summary of the invention

Accordingly the present invention provides an improved process for the production of benzaldehyde with 40-50% selectivity comprising carrying out catalytic liquid phase air oxidation of toluene by providing a continuous flow of air in the presence of a catalyst selected from the group consisting of Fe, Co, Mn, Mo and Ni, a co-catalyst selected from manganese and copper salts, a promoter and bromine source selected from cobalt bromide, sodium bromide and zinc bromide, and a carboxylic acid solvent selected from the group consisting of acetic, propionic, and benzoic acid ranging between 0.05 to 0.3 wt. times with respect to toluene, at a temperature ranging between 60-130°C and pressure in the range of 1-10 bars (1×10⁵-1×10⁶N.m⁻²) for a period of 0.5-1.5 hours to obtain benzaldehyde (40-50%) along with other by-products.

In perferred embodiments, the invention permits the production of benzaldehyde with 40-50% selectivity by catalytic liquid phase air oxidation of toluene which comprises the production of value added products benzaldehyde (40-50%), benzyl alcohol (5-10%) and benzoic acid in large proportions without any trace amounts of benzyl bromide while maintaining transformation percentage <25% in the catalytic liquid phase air oxidation of toluene with continuous flow of air employing the salts of Fe, Co, Mn, Mo, Ni as catalysts, manganese or copper salts as co-catalysts and cobalt bromide, sodium bromide and zinc bromide as promoters as well as bromine source and low content of carboxylic acid as solvent such as acetic, propionic, benzoic acids 0.05 to 0.3 wt. times with respect to toluene, in the temperatures 60-130°C and pressures in the range of 1- 10 bars (1×10⁵-1×10⁶N.m⁻²) for a period of 0.5-1.5 hrs.

In an embodiment of the present invention the conversion is restricted to 15- 25%.

In another embodiment of the present invention the process is a catalytic liquid phase reaction.

In still another embodiment of the present invention the selectivity to benzyl alcohol is 5-10%.

In still another embodiment of the present invention air is used as the oxidant.

In still another embodiment of the present invention the catalyst contains a heavy metallic compound such as cobalt, molybdenum or iron as catalyst. The concentration of these salts with respect to toluene in the reaction system is very low in the range of 0.07-0.28 mol%.

In still another embodiment of the present invention the co-catalyst contains a heavy metallic compound such as manganese or copper and concentration with respect to toluene is very low in the range of 0.004 -0.017 mol%.

In still another embodiment of the present invention the catalyst and co-catalyst are organic acid salts selected from the group consisting of formic acid salts, acetic acid salts, propionic acid salts, etc. of the heavy metallic compound.

In still another embodiment of the present invention the promoter is a bromine compound such as cobalt, sodium or zinc bromide. The concentration of the promoters in the reaction system with respect to the toluene is very low in the range of 0.14 - 1.14 mol%.

In still another embodiment of the present invention the atomic ratio of zinc atoms in the promoter with respect to heavy metallic atoms in the catalyst is in the range of 0.05 - 8.

In still another embodiment of the present invention in recycle reactions conducted with the recovered catalyst, near identical conversions and selectivities were obtained without any addition of promoter or bromide on each recycle.

In still another embodiment of the present invention in recycle reactions conducted, the consumption of promoter is minimised.

In still another embodiment of the present invention the process is conducted with low concentration of catalyst, co-catalyst and promoter in order to slow down corrosion of the reactor.

In still another embodiment of the present invention the turnover number is in the range of 350-700.

In still another embodiment of the present invention the process totally eliminates the formation of benzyl bromide as a by-product.

In still another embodiment of the present invention the benzaldehyde produced is chlorine free and can be used for a wide range of applications.

The process also results in the production of value added byproducts benzyl alcohol (5-10%) and benzoic acid in large proportions without any trace amounts of benzyl bromide while maintaining transformation percentage <25%.

It has now been discovered that there is a high selectivity of benzaldehyde and benzyl alcohols in the air oxidation of toluene, with turnover number 350-700. The low concentration of solvent, carboxylic acid in the reaction minimise the release of HBr, which in turn generates free radical Br on interaction with Co³⁺ sufficient to the initiation of the reaction. In a recycle reaction conducted with the recovered catalyst, near identical conversions and selectivities were obtained without any addition of promoter or bromide. This result clearly establishes that bromine consumption in the system is minimised. An analysis of the results of experiments provides confirmation of the reduced consumption of bromine in the system. Therefore, formation of the by-product, benzyl bromide which requires higher content of HBr is eliminated, thus, resulting in production of benzaldehyde which meets specification of the food grade. This also helps to achieve higher turnover number by allowing optimum use of catalyst and co-catalyst. Apart from this, low concentration of catalyst, co-catalyst and promoter further slows down corrosion on the reactor, a very important factor affecting the economics of process. The process is environmentally safe since there is no effluent disposal problem.

The following examples are given by way of illustration of the present invention and therefore should not be construed to limit the scope of the present invention.

### Comparative Example 1

Buchi glass autoclave of 500ml capacity equipped with a gas connecting tube, stirrer and pressure gauge was deployed. The following materials were taken in the reaction vessel - 99.6ml of toluene, 200.4ml of acetic acid, 0.75g of cobalt acetate.4 water salt, 2.5g of sodium bromide. After flushing thrice with air, the solution was heated slowly up to 110°C while stirring the reaction mixture. After system attained a constant temperature of 110°C, it was pressurised with air to 9Kg/cm² with 2Lt/min out flow. After 2 hrs, the reaction mixture was taken out. Gas chromatographic and titrimetric analysis on the product indicates conversion of 20.43% with selectivity towards benzaldehyde 40.98%, benzyl alcohol 0.79%, benzoic acid 58.24%.

### Example 2

Buchi glass autoclave of 500ml capacity equipped with a gas connecting tube, stirrer and pressure gauge was deployed. The following materials were taken in the reaction vessel; 107ml of toluene, 193ml of acetic acid, 5g of cobalt acetate.4 water salt, 0.3g of manganese acetate.4 water salt, 9g of zinc bromide. After flushing thrice with air, the solution was heated slowly up to 80°C while stirring the reaction mixture. After system attained a constant temperature of 80°C, it was pressurised with air to 9Kg/cm² with 2Lt/min out flow. After 1.15hrs, the reaction mixture was taken out. Gas chromatographic and titrimetric analysis on the product indicates conversion of 13.2 % with selectivity towards benzaldehyde 62.77%, benzyl alcohol 2.75 % and benzoic acid 34.46%.

### Comparative Example 3

Buchi glass autoclave of 500ml capacity equipped with a gas connecting tube, stirrer and pressure gauge was deployed. The following materials were taken in the reaction vessel - 150ml of toluene, 150ml of acetic acid, 1.5g of cobalt acetate.4 water salt, 5g of sodium bromide. After flushing thrice with air, the solution was heated slowly up to 110°C while stirring the reaction mixture. After system attained a constant temperature of 110°C, it was pressurised with air to 9Kg/cm² with 2Lt/min out flow. After 2.15hrs, the reaction mixture was taken out. Gas chromatographic and titrimetric analysis on the product indicates conversion of 15.17% with selectivity towards benzaldehyde 49.95%, benzyl alcohol 3.25% and benzoic acid 46.8%.

### Comparative Example 4

Buchi glass autoclave of 500ml capacity equipped with a gas connecting tube, stirrer and pressure gauge was deployed. The following materials were taken in the reaction vessel - 280ml of toluene, 20ml of acetic acid, 1.5g of cobalt acetate.4 water salt, 5g of sodium bromide. After flushing thrice with air, the solution was heated slowly up to 110°C while stirring the reaction mixture. After system attained a constant temperature of 110°C, it was pressurised with air to 9Kg/cm² with 2Lt/min out flow. After 2.15hrs, the reaction mixture was taken out. Gas chromatographic and titrimetric analysis on the product indicates conversion of 9.22% with selectivity towards benzaldehyde 46.23%, benzyl alcohol 10.25%, benzoic acid 43.51%.

### Comparative Example 5

Buchi glass autoclave of 500ml capacity equipped with a gas connecting tube, stirrer and pressure gauge was deployed. The following materials were taken in the reaction vessel - 224.3ml of toluene, 75.7ml of acetic acid, 1.5g of cobalt acetate.4 water salt, 5g of sodium bromide. After flushing thrice with air, the solution was heated slowly up to 110°C while stirring the reaction mixture. After system attained a constant temperature of 110°C, it was pressurised with air to 9Kg/cm² with 2Lt/min out flow. After 2.15hrs, the reaction mixture was taken out. Gas chromatographic and titrimetric analysis on the product indicates conversion of 17.4% with selectivity towards benzaldehyde 43.48%, benzyl alcohol 4.37% and benzoic acid 52.15%.

### Comparative Example 6

Buchi glass autoclave of 500ml capacity equipped with a gas connecting tube, stirrer and pressure gauge was deployed. The following materials were taken in the reaction vessel; 224.3ml of toluene, 75.7ml of acetic acid, 1.5g of cobalt acetate.4 water salt, 0.09g of manganese acetate 4 water salt, 5.4g of zinc bromide. After flushing thrice with air, the solution was heated slowly up to 80°C while stirring the reaction mixture. After system attained a constant temperature of 80°C, it was pressurised with air to 9Kg/cm² with 2Lt/min out flow. After 2hrs, the reaction mixture was taken out. Gas chromatographic and titrimetric analysis on the product indicates conversion of 10.32% with selectivity towards benzaldehyde 59.47%, benzyl alcohol 6.23% and benzoic acid 34.29%.

### Example 7

Buchi glass autoclave of 500ml capacity equipped with a gas connecting tube, stirrer and pressure gauge was deployed. The following materials were taken in the reaction vessel; 225.3ml of toluene, 75.7ml of acetic acid, 1.5g of cobalt acetate.4 water salt, 0.09g of manganese acetate.4 water salt, 2.7g of zinc bromide. After flushing thrice with air, the solution was heated slowly up to 110°C while stirring the reaction mixture. After system attained a constant temperature of 110°C, it was pressurised with air to 9Kg/cm² with 2Lt/min out flow. After 1.45hrs, the reaction mixture was taken out. Gas chromatographic and titrimetric analysis on the product indicates conversion of 16.36% with selectivity towards benzaldehyde 39.6%, benzyl alcohol 5.53%, and benzoic acid 54.8%.

### Example 8

Buchi glass autoclave of 500ml capacity equipped with a gas connecting tube, stirrer and pressure gauge. The following materials were taken in the reaction vessel; 224.3 ml of toluene, 75.7ml of acetic acid, 1.12g of cobalt acetate.4 water salt, 0.06g of manganese acetate.4 water salt, 2.02g of zinc bromide. After flushing thrice with air, the solution was heated slowly upto 110°C while stirring the reaction mixture. After system attained a constant temperature of 110°C, it was pressurised with air to 9Kg/cm² with 2Lt/min out flow. After 1.15hrs, the reaction mixture was taken out. Gas chromatographic and titrimetric analysis on the product indicates conversion of 11.06% with selectivity towards benzaldehyde 39.94%, benzyl alcohol 8.9%, benzoic acid 51.14%.

### Example 9

Buchi glass autoclave of 500ml capacity equipped with a gas connecting tube, stirrer and pressure gauge was deployed. The following materials were taken in the reaction vessel; 224.3ml of toluene, 75.7ml of acetic acid, 1.12g of cobalt acetate.4 water salt, 0.06g of manganese acetate.4 water salt, 1.01g of zinc bromide. After flushing thrice with air, the solution was heated slowly upto 110°C while stirring the reaction mixture. After system attained a constant temperature of 110°C, it was pressurised with air to 9Kg/cm² with 2Lt/min out flow. After 1.15hrs, the reaction mixture was taken out. Gas chromatographic and titrimetric analysis on the product indicates conversion of 14.22% with selectivity towards benzaldehyde 36.40%, benzyl alcohol 8.91% and benzoic acid 54.68%.

### Example 10

### Catalyst Recycle

The reaction mixture of example 9 containing toluene, acetic acid, benzaldehyde, benzoic acid, benzyl alcohol and catalyst mixture was taken for recycle study. Water was then added to the reaction mixture while it is hot after recovering toluene and acetic acid by distillation and then cooled to room temperature. Benzoic acid formed was removed by filtration and the remaining organic and aqueous layers were separated. Organic layer was subjected to distillation to get benzaldehyde and benzyl alcohol and the aqueous layer is concentrated to get the catalyst composite mixture cake.

Buchi glass autoclave of 500ml capacity equipped with a gas connecting tube, stirrer and pressure gauge was deployed. The following materials were taken in the reaction vessel; 224.3ml of toluene, 75.7ml of acetic acid, recovered composite mixture cake containing cobalt acetate, manganese acetate and zinc bromide. After flushing thrice with air, the solution was heated slowly upto 110°C while stirring the reaction mixture. After system attained a constant temperature of 10°C, it was pressurised with air to 9Kg/cm² with 2Lt/min out flow. After 1.15hrs, the reaction mixture was taken out. Gas chromatographic and titrimetric analysis on the product indicates conversion of 11.42% with selectivity towards benzaldehyde 35.40 %, benzyl alcohol 11.39% and benzoic acid 53.18%.

### Comparative Example 11

Buchi glass autoclave of 500ml capacity equipped with a gas connecting tube, stirrer and pressure gauge was deployed. The following materials were taken in the reaction vessel - 224.3ml of toluene, 75.7ml acetic acid, 0.75g of cobalt acetate.4 water salt, 0.04g of manganese acetate.4 water salt, 1.35g of zinc bromide. After flushing thrice with air, the solution was heated slowly up to 110°C while stirring the reaction mixture. After system attained a constant temperature of 110°C, it was pressurised with air to 9Kg/cm² with 2Lt/min out flow. After 2hrs, the reaction mixture was taken out. Gas chromatographic and titrimetric analysis on the product indicates conversion of 12.75% with selectivity towards benzaldehyde 44.71%, benzyl alcohol 5.15% and benzoic acid 50.1%.

### Comparative Example 12

Buchi glass autoclave of 500ml capacity equipped with a gas connecting tube, stirrer and pressure gauge was deployed. The following materials were taken in the reaction vessel; 224.3ml of toluene, 75.7ml of acetic acid, 0.375g of cobalt acetate 4 water salt, 0.0225g of manganese acetate .4 water salt, 0.675g of zinc bromide. After flushing thrice with air, the solution was heated slowly up to 130°C while stirring the reaction mixture. After system attained a constant temperature of 130°C, it was pressurised with air to 9Kg/cm² with 2Lt/min out flow. After 2.30hrs, the reaction mixture was taken out. Gas chromatographic and titrimetric analysis on the product indicates conversion of 10.60% with selectivity towards benzaldehyde of 40.32%, benzyl alcohol 3.19%, benzoic acid 56.40%.

### Example 13

Buchi glass autoclave of 500ml capacity equipped with a gas connecting tube, stirrer and pressure gauge was deployed. The following materials were taken in the reaction vessel; 260ml of toluene, 46g of benzoic acid, 1.5g of cobalt acetate.4 water salt, 0.09g of manganese acetate.4 water salt, 2.7g of zinc bromide. After flushing thrice with air, the solution was heated slowly upto 130°C while stirring the reaction mixture. After system attained a constant temperature of 130°C, it was pressurised with air to 9Kg/cm² with 2Lt/min out flow. After 1hr, the reaction mixture was taken out. Gas chromatographic and titrimetric analysis on the product indicates conversion of 8.69% with selectivity towards benzaldehyde of 39.97%, benzyl alcohol 7.99%, benzoic acid 51.84%.

### Example 14

Buchi glass autoclave of 500ml capacity equipped with a gas connecting tube, stirrer and pressure gauge was deployed. The following materials were taken in the reaction vessel; 280ml of toluene, 25.52g of benzoic acid, 1.5g of cobalt acetate.4 water salt, 0.09g of manganese acetate.4 water salt, 2.7g of zinc bromide. After flushing thrice with air, the solution was heated slowly up to 130°C while stirring the reaction mixture. After system attained a constant temperature of 130°C, it was pressurised with air to 9Kg/cm² with 2Lt/min out flow. After 1.30hr, the reaction mixture was taken out. Gas chromatographic and titrimetric analysis on the product indicates conversion of 6.38% with selectivity towards benzaldehyde of 39.5%, benzyl alcohol 4.9% and benzoic acid 55.44%.

### Example 15

Buchi glass autoclave of 500ml capacity equipped with a gas connecting tube, stirrer and pressure gauge was deployed. The following materials were taken in the reaction vessel; 280ml of toluene, 12.76g of benzoic acid, 1.5g of cobalt acetate.4 water salt, 0.09g of manganese acetate.4 water salt and 2.7g of zinc bromide. After flushing thrice with air, the solution was heated slowly upto 130°C while stirring the reaction mixture. After system attained a constant temperature of 130°C, it was pressurised with air to 9Kg/cm² with 2Lt/min out flow. After 1.30hr, the reaction mixture was taken out. Gas chromatographic and titrimetric analysis on the product indicates conversion of 6.79% with selectivity towards benzaldehyde of 36.2%, benzyl alcohol 8.3%, and benzoic acid 55.4%.

### Example 16

Buchi glass autoclave of 500ml capacity equipped with a gas connecting tube, stirrer and pressure gauge was deployed. The following materials were taken in the reaction vessel; 260ml of toluene, 46g of benzoic acid, 3ml of water, 0.75g of cobalt acetate.4 water salt, 0.04g of manganese acetate.4 water salt and 1.35g of zinc bromide. After flushing thrice with air, the solution was heated slowly upto 130°C while stirring the reaction mixture. After system attained a constant temperature of 130°C, it was pressurised with air to 9Kg/cm² with 2Lt/min out flow. After 1.30hr, the reaction mixture was taken out. Gas chromatographic and titrimetric analysis on the product indicates conversion of 4.7% with selectivity towards benzaldehyde of 36.11%, benzyl alcohol 8.14%, benzoic acid 55.48%.

### Example 17

Buchi glass autoclave of 500ml capacity equipped with a gas connecting tube, stirrer and pressure gauge was deployed. The following materials were taken in the reaction vessel; 260ml of toluene, 46g of benzoic acid, 3ml of water, 1.5g of cobalt acetate.4 water salt, 0.09g of manganese acetate.4 water salt and 2.7g of zinc bromide. After flushing thrice with air, the solution was heated slowly upto 130°C while stirring the reaction mixture. After system attained a constant temperature of 130°C, it was pressurised with air to 9Kg/cm² with 2Lt/min out flow. After 1.30hr, the reaction mixture was taken out. Gas chromatographic and titrimetric analysis on the product indicates conversion of 14.86% with selectivity towards benzaldehyde of 24.28%, benzyl alcohol 2.00%, benzoic acid 73.72%.

The results are given in Table 1.

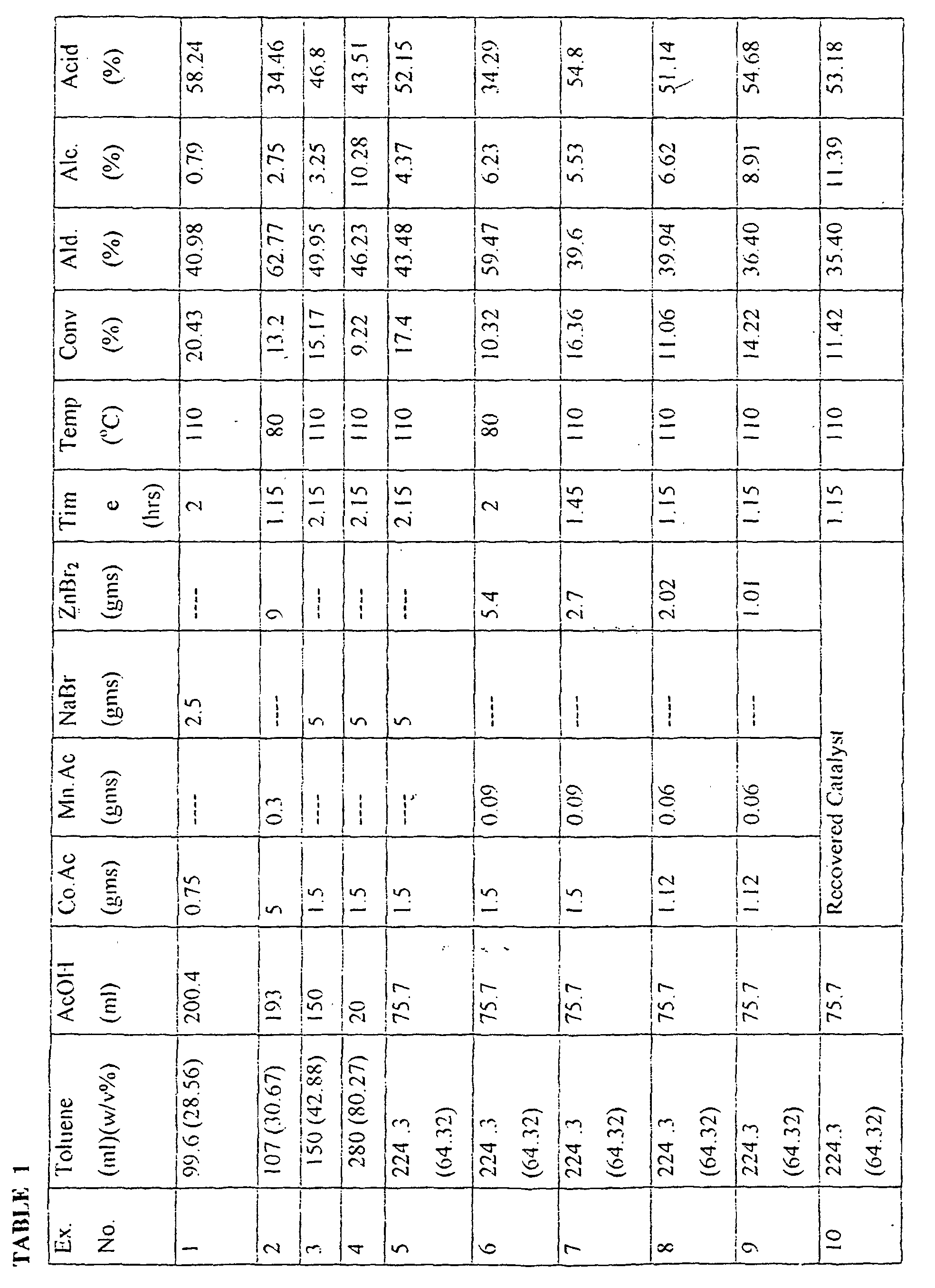

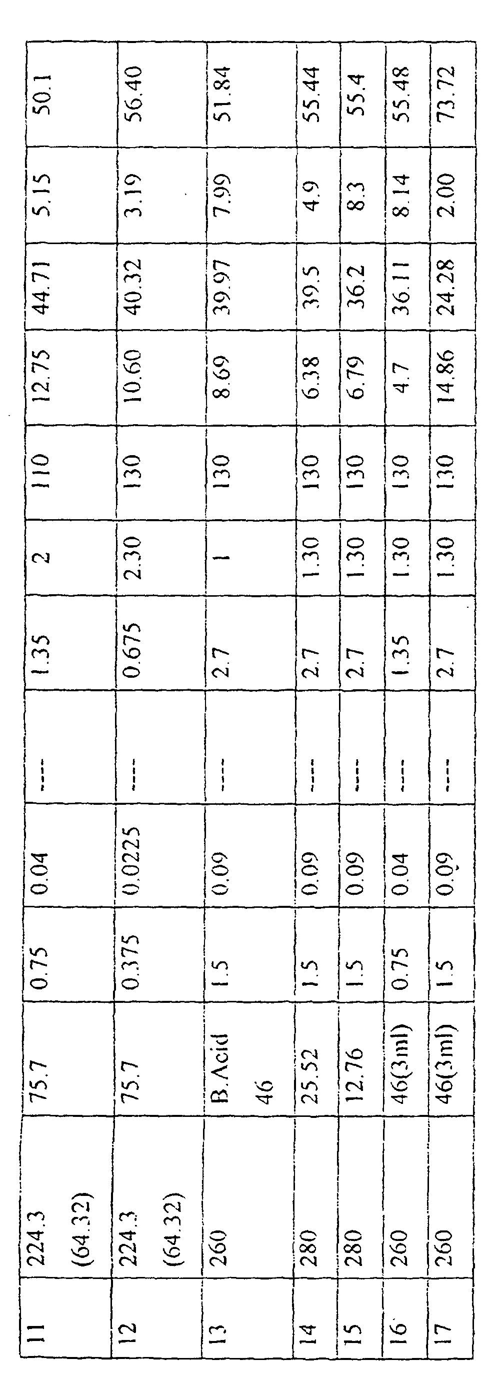

The main advantages of the present invention are:
1. The process is a catalytic liquid phase reaction.
2. The selectivity for benzaldehyde obtained in this process is in the range of 40-50%.
3. The selectivity for benzyl alcohol obtained in this process is in the range of 5-10%.
4. Low concentration of catalyst, co-catalyst and promoter is used
5. The temperatures and pressures employed in the reaction are moderate.
6. The residence time in the reactor is short.
7. Acetic acid is used as the solvent and the amount used is in the range of 0.05 to 0.3 wt. times with respect to toluene.
8. An eco-friendly process for the production of benzaldehyde without the formation of benzyl bromide was developed.
9. Benzaldehyde produced is free from chlorine and can be used over a wide range of applications.
10. High turnover number 350-700 is realised for the first time.
11. The catalytic system afforded recyclability without any addition of promoter thereby utilisation of the promoter in the process on each recycle is minimised.
12. Employment of the low concentration catalytic system slows down corrosion on the reactor, a very important factor affecting economics of the project.
13. The present process is environmentally safe since there is no effluent disposal problem.
14. The process is economical.

## Claims

1. An improved process for the production of benzaldehyde with 40-50% selectivity comprising carrying out catalytic liquid phase air oxidation of toluene by providing a continuous flow of air in the presence of a catalyst selected from the group consisting of salts of Fe, Co, Mn, Mo and Ni, a co-catalyst selected from the group consisting of manganese and copper salts, a promoter and bromine source selected from cobalt bromide, sodium bromide and zinc bromide, and a carboxylic acid solvent selected from the group consisting of acetic, propionic and benzoic acid ranging between 0.05 to 0.3 wt. times with respect to toluene, at a temperature ranging between 60-130°C and pressure in the range of 1-10 bars (1 × 10⁵ - 1 × 106 N.m⁻²) for a period of 0.5-1.5 hours to obtain benzaldehyde (40-50%) along with other by-products.

2. A process as claimed in claim 1 wherein the by-products obtained comprise benzyl alcohol in an amount of 5-10% and benzoic acid.

3. A process as claimed in claim 1 or claim 2, wherein the promoter is selected from the group consisting of cobalt bromide, sodium bromide and zinc bromide.

4. A process as claimed in any of claims 1 to 3, wherein the turnover number is 350-700.

5. A process as claimed in any preceding claim, wherein the recovered catalyst is recycled into the reaction system without any addition of promoter/bromine source.

6. A process as claimed in any preceding claim, wherein the catalyst contains a heavy metallic compound selected from cobalt, molybdenum and iron.

7. A process as claimed in any preceding claim, wherein the concentration of the catalyst with respect to toluene in the reaction system is in the range of 0.07-0.28 mol %.

8. A process as claimed in any preceding claim, wherein the co-catalyst contains a heavy metallic compound selected from manganese and copper and concentration of said co-catalyst with respect to toluene is in the range of 0.004 -0.017 mol %.

9. A process as claimed in any preceding claim, wherein the catalyst and co-catalyst are selected from the group consisting of organic acid salts such as formic acid salts, acetic acid salts, propionic acid salts of said heavy metallic compound.

10. A process as claimed in any preceding claim, wherein the concentration of the promoter in the reaction system with respect to the toluene is in the range of 0.14 - 1.14 mol % and wherein the promoter is zinc bromide and the atomic ratio of zinc atoms in the promoter with respect to heavy metallic atoms in the catalyst is in the range of 0.05-8.

## Patentansprüche

1. Verbessertes Verfahren zur Herstellung von Benzaldehyd mit einer Selektivität von 40-50%, bei dem man eine katalytische Luftoxidation von Toluol in der Flüssigphase durchführt, indem man in Gegenwart eines Katalysators aus der Gruppe bestehend aus Fe-, Co-, Mn-, Mo- und Ni-Salzen, eines Cokatalysators aus der Gruppe bestehend aus Mangan- und Kupfersalzen, einer als Promotor und Bromquelle dienenden, unter Cobaltbromid, Natriumbromid und Zinkbromid ausgewählten Substanz und eines Carbonsäurelösungsmittels aus der Gruppe bestehend aus Essigsäure, Propionsäure und Benzoesäure im Bereich vom gewichtsbezogenen 0,05- bis 0,3-fachen, bezogen auf Toluol, bei einer Temperatur im Bereich von 60 bis 130°C und einem Druck im Bereich von 1-10 bar (1 x 10⁵ bis 1 x 10⁶ N.m⁻²) über einen Zeitraum von 0,5 bis 1,5 Stunden einen kontinuierlichen Luftstrom bereitstellt, wobei man Benzaldehyd (40-50%) zusammen mit anderen Nebenprodukten erhält.

2. Verfahren nach Anspruch 1, bei dem die erhaltenen Nebenprodukte Benzylalkohol in einer Menge von 5-10% und Benzoesäure umfassen.

3. Verfahren nach Anspruch 1 oder 2, bei dem man den Promotor aus der Gruppe bestehend aus Cobaltbromid, Natriumbromid und Zinkbromid auswählt.

4. Verfahren nach einem der Ansprüche 1 bis 3, bei dem die Umsatzzahl 350 bis 700 beträgt.

5. Verfahren nach einem der vorhergehenden Ansprüche, bei dem man den zurückgewonnenen Katalysator ohne Zugabe von Promotor/Bromquelle in das Reaktionssystem zurückführt.

6. Verfahren nach einem der vorhergehenden Ansprüche, bei dem der Katalysator eine unter Cobalt, Molybdän und Eisen ausgewählte Schwermetallverbindung enthält.

7. Verfahren nach einem der vorhergehenden Ansprüche, bei dem die Konzentration des Katalysators, bezogen auf Toluol im Reaktionssystem, im Bereich von 0,07 bis 0,28 Mol-% liegt.

8. Verfahren nach einem der vorhergehenden Ansprüche, bei dem der Cokatalysator eine unter Mangan und Kupfer ausgewählte Schwermetallverbindung enthält und die Konzentration des Cokatalysators, bezogen auf Toluol im Reaktionssystem, im Bereich von 0,004 bis 0,017 Mol-% liegt.

9. Verfahren nach einem der vorhergehenden Ansprüche, bei dem man den Katalysator und Cokatalysator aus der Gruppe bestehend aus Salzen organischer Säuren wie Ameisensäuresalzen, Essigsäuresalzen und Propionsäuresalzen der Schwermetallverbindung auswählt.

10. Verfahren nach einem der vorhergehenden Ansprüche, bei dem die Konzentration des Promotors im Reaktionssystem, bezogen auf das Toluol, im Bereich von 0,14 bis 1,14 Mol-% liegt, es sich bei dem Promotor um Zinkbromid handelt und das Atomverhältnis von Zinkatomen im Promotor zu Schwermetallatomen im Katalysator im Bereich von 0,05 bis 8 liegt.

## Revendications

1. Procédé amélioré de production de benzaldéhyde avec une sélectivité de 40 à 50 %, comprenant la mise en oeuvre d'une oxydation catalytique à l'air en phase liquide du toluène en procurant un flux continu d'air en présence d'un catalyseur choisi parmi le groupe constitué de sels de Fe, Co, Mn, Mo et Ni, d'un co-catalyseur choisi parmi le groupe constitué de sels de manganèse et de cuivre, d'un activateur et d'une source de brome choisie parmi le bromure de cobalt, le bromure de sodium et le bromure de zinc, et d'un solvant acide carboxylique choisi parmi le groupe constitué de l'acide acétique, de l'acide propionique et de l'acide benzoïque, dont le facteur pondéral s'échelonne entre 0,05 et 0,3 par rapport au toluène, à une température s'échelonnant entre 60 et 130°C et une pression dans la plage de 1 à 10 bars (1 x 10⁵ - 1 x 10⁶ N.m⁻²) pendant une période de 0,5 à 1,5 heure en vue d'obtenir du benzaldéhyde (de 40 à 50 %) conjointement avec d'autres produits secondaires.

2. Procédé selon la revendication 1, dans lequel les produits secondaires obtenus comprennent de l'alcool benzylique en une quantité de 5 à 10 % et de l'acide benzoïque.

3. Procédé selon la revendication 1 ou la revendication 2, dans lequel l'activateur est choisi parmi le groupe constitué du bromure de cobalt, du bromure de sodium et du bromure de zinc.

4. Procédé selon l'une quelconque des revendications 1 à 3, dans lequel l'indice de renouvellement est de 350 à 700.

5. Procédé selon l'une quelconque des revendications précédentes, dans lequel le catalyseur récupéré est recyclé dans le système réactionnel sans aucune addition d'activateur/source de brome.

6. Procédé selon l'une quelconque des revendications précédentes dans lequel le catalyseur contient un composé métallique lourd choisi parmi le cobalt, le molybdène et le fer.

7. Procédé selon l'une quelconque des revendications précédentes, dans lequel la concentration du catalyseur par rapport au toluène dans le système réactionnel est dans la plage de 0,07 à 0,28 % en moles.

8. Procédé selon l'une quelconque des revendications précédentes dans lequel le co-catalyseur contient un composé métallique lourd choisi parmi le manganèse et le cuivre et la concentration dudit co-catalyseur par rapport au toluène est dans la plage de 0,004 à 0,017 % en moles.

9. Procédé selon l'une quelconque des revendications précédentes, dans lequel le catalyseur et le co-catalyseur sont choisis parmi le groupe constitué de sels d'acides organiques tels que des sels d'acide formique, des sels d'acide acétique, des sels d'acide propionique dudit composé métallique lourd.

10. Procédé selon l'une quelconque des revendications précédentes, dans lequel la concentration de l'activateur dans le système réactionnel par rapport au toluène est dans la plage de 0,14 à 1,14 % en moles et dans lequel l'activateur est le bromure de zinc et le rapport atomique d'atomes de zinc dans l'activateur par rapport aux atomes métalliques lourds dans le catalyseur est dans la plage de 0,05 à 8.
